(19) Europäisches Patentamt
European Patent Office
Office européen des brevets

(11) Veröffentlichungsnummer: **0 061 056**
**A1**

(12) # EUROPÄISCHE PATENTANMELDUNG

(21) Anmeldenummer: 82101812.4

(22) Anmeldetag: 08.03.82

(51) Int. Cl.³: C 07 D 471/04
A 61 K 31/44
//C07D207/40, (C07D471/04, 221/00, 209/00)

(30) Priorität: 21.03.81 DE 3111155

(43) Veröffentlichungstag der Anmeldung: 29.09.82 Patentblatt 82/39

(84) Benannte Vertragsstaaten: AT BE CH DE FR GB IT LI NL SE

(71) Anmelder: Troponwerke GmbH & Co. KG
Berliner Strasse 156
D-5000 Köln 80(DE)

(72) Erfinder: Jacobi, Haireddin, Dr.
Finkenweg 2
D-5653 Leichlingen(DE)

(72) Erfinder: Eger, Kurt, Dr.
Dohmstrasse 2
D-5300 Bonn(DE)

(72) Erfinder: Roth, Hermann Josef, Prof. Dr.
Boeckingstrasse 4
D-5340 Bad Honnef(DE)

(72) Erfinder: Zimmermann, Werner, Dr.
Am Klostergarten 3
D-5300 Bonn(DE)

(74) Vertreter: Senftl, Hannes, Dr. et al,
c/o Bayer AG Zentralbereich Patente Marken und Lizenzen
D-5090 Leverkusen 1, Bayerwerk(DE)

(54) **5,6-Dimethyl-pyrrolo(2,3-b)pyridine, Verfahren zu ihrer Herstellung und ihre Verwendung als Arzneimittel.**

(57) Die vorliegende Erfindung betrifft neue 5,6-Dimethylpyrrolo[2,3-b]pyridine der allgemeinen Formel I

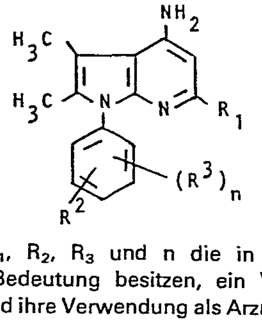

in welcher $R_1$, $R_2$, $R_3$ und n die in der Beschreibung angegebene Bedeutung besitzen, ein Verfahren zu ihrer Herstellung und ihre Verwendung als Arzneimittel, insbesondere als Antipholgistika und als Mittel zur Behandlung des zentralen Nervensystems.

EP 0 061 056 A1

0061056

TROPONWERKE GmbH & Co. KG 5000 Köln 80

KS/bc/c
Ia (Pha)

5,6-Dimethyl-pyrrolo[2,3-b]pyridine, Verfahren zu ihrer Herstellung und ihre Verwendung als Arzneimittel

Die vorliegende Erfindung betrifft neue 5,6-Dimethyl-pyrrolo[2,3-b]pyridine, ein Verfahren zu ihrer Herstellung und ihre Verwendung als Arzneimittel, insbesondere als Antiphlogistika und als Mittel zur Behandlung des zentralen Nervensystems.

Ähnliche Pyrrolo[2,3-b]pyridin-Derivate sind bereits bekannt geworden, z.B. als Inhibitoren des pflanzlichen Wachstumshormons 2-Indolylessigsäure (J.M. Webster, Nematologica 13, 256 (1967)), oder als Verbindungen, die das Wachstum von Gram-positiven Bakterien hemmen (M. Hooper, D.A. Patterson und D.G. Wibberley, J.Pharm. Pharmacol. 17, 734 (1965)).

Weiterhin sind Verbindungen bekannt, die antiinflammatorische, analgetische und antipyretische Aktivität zeigen (Merck & Co., Netherland Patent 6 510 648 (1966), ref. in C.A. 65, 13711 (1966)).

Stoffe aus dieser Verbindungsklasse haben jedoch bisher noch keinen Eingang in die Humanmedizin gefunden.

Die Erfindung betrifft neue 5,6-Dimethyl-pyrrolo[2,3-b]-pyridine der allgemeinen Formel (I)

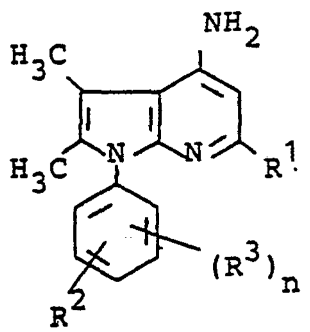

(I)

in welcher

$R_1$ für Wasserstoff oder niederes Alkyl steht,

$R_2$ für Wasserstoff, Halogen, Nitro, niederes Alkyl oder Alkoxy, Trifluormethyl oder Dimethylamino steht,

$R_3$ jeweils für Wasserstoff, Halogen oder niederes Alkyl oder Alkoxy steht und

n für 1 oder 2 steht,

sowie ihre physiologisch unbedenklichen Additionssalze.

Die Erfindung betrifft auch ein Verfahren zur Herstellung der neuen 5,6-Dimethyl-pyrrolo[2,3-b]pyridine der allgemeinen Formel (I), in welcher $R_1$, $R_2$, $R_3$ und n die oben angeführte Bedeutung haben, welches dadurch gekennzeichnet ist, daß man Pyrrol-Derivate der allgemeinen Formel (II)

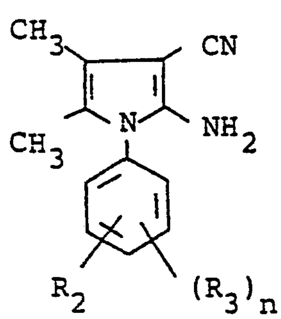

(II)

mit Diketoverbindungen der allgemeinen Formel (III)

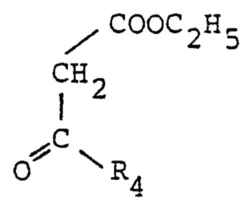

(III)

in welcher

$R_4$ für die Methyl- oder die Ethoxycarbonylgruppe steht,

in Gegenwart von p-Toluolsulfonsäure als Katalysator und in Gegenwart von inert-organischen Lösungsmitteln bei erhöhter Temperatur zu Verbindungen der allgemeinen Formel (IV) umsetzt,

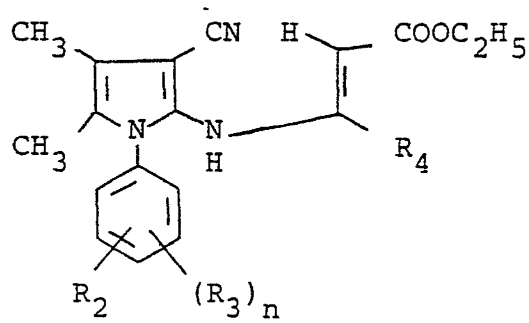

(IV)

in welcher

$R_2$, $R_3$, $R_4$ und n die oben angeführte Bedeutung haben,

und die Verbindung der allgemeinen Formel (IV) anschließend in Gegenwart von organischen Lösungsmitteln und gegebenenfalls in Gegenwart von Katalysatoren bei erhöhter Temperatur zu Verbindungen der Formel (V)

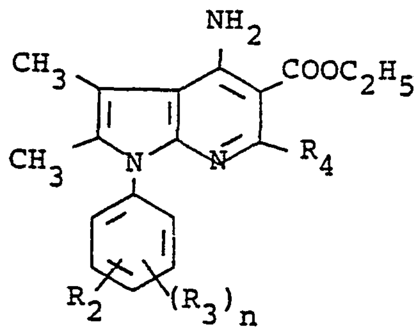

(V)

cyclisiert und diese Verbindungen anschließend in Gegenwart von alkoholischer Kalilauge und gegebenenfalls in Gegenwart von inerten Lösungsmitteln bei erhöhter Temperatur und anschließender Zufügung von Säuren zu Verbindungen der allgemeinen Formel (VI)

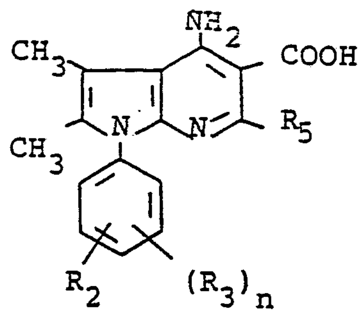

(VI)

in welcher

$R_2$,$R_3$ und n die oben angegebene Bedeutung haben,

$R_5$ für eine Methyl- oder Carboxylgruppe steht,

umsetzt, und anschließend die Verbindungen der allgemeinen Formel (VI) in Gegenwart von 85 %iger Phosphorsäure über einen kürzeren Zeitraum bei erhöhter Temperatur zu Verbindungen der allgemeinen Formel (VII)

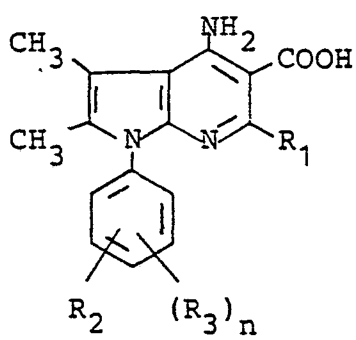

(VII)

TP 39

in welcher
$R_1, R_2, R_3$ und n die oben angegebene Bedeutung haben,

umsetzt und diese Verbindungen der allgemeinen Formel (VII) dann in Gegenwart von 85 %iger Phosphorsäure über einen längeren Zeitraum bei erhöhter Temperatur zu Verbindungen der allgemeinen Formel (I) decarboxyliert.

Die Herstellung der neuen Pyrrolo/2,3-b/pyridine kann durch folgendes Reaktionsschema wiedergegeben werden:

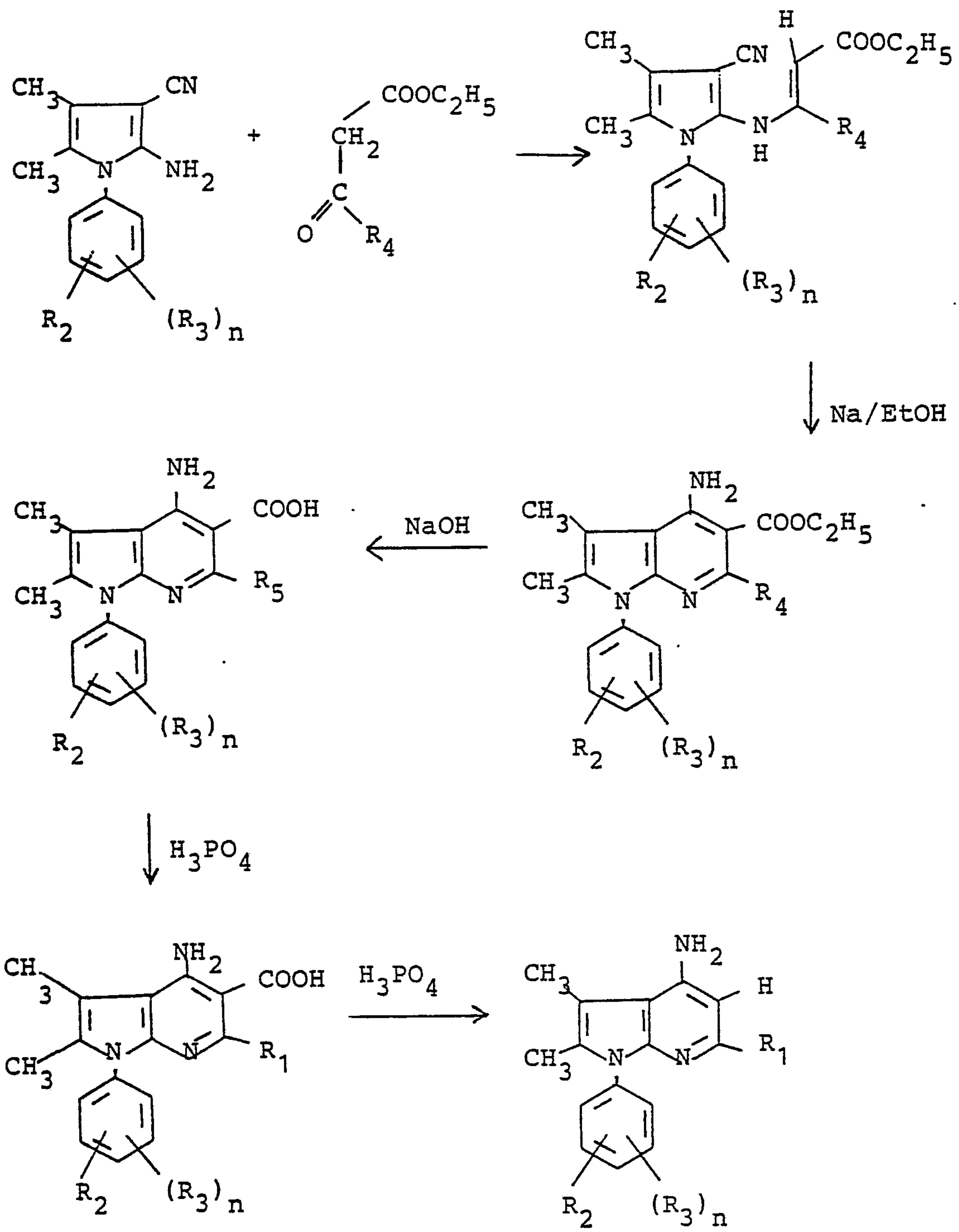
Na/EtOH
NaOH
$H_3PO_4$
$H_3PO_4$

Die erfindungsgemäßen Verbindungen der Formel (I) können mit geeigneten Säuren in physiologisch unbedenkliche Säureadditionssalze überführt werden. Die erfindungsgemäßen Verbindungen der Formel (VII) können ebenso mit geeigneten Basen in physiologisch unbedenkliche Basenadditionssalze überführt werden. Die Salze besitzen die gleichen vorteilhaften Eigenschaften wie die freien Verbindungen.

Als geeignete Säuren seien genannt:

Salzsäure, Milchsäure, Weinsäure.

Als geeignete Basen seien genannt:

NaOH, KOH.

In den allgemeinen Formeln bedeutet:

Halogen vorzugsweise Chlor, Brom oder Fluor,

niederes Alkyl, vorzugsweise Alkyl mit 1 bis 4 Kohlenstoffatomen; im einzelnen seien genannt Methyl-, Ethyl-, Propyl-, Isopropyl-, Butyl-, Isobutyl- oder tertiäres Butyl-; besonders bevorzugt ist Methyl;

niederes Alkoxy, vorzugsweise Methoxy, Ethoxy, Propoxy oder Isopropoxy, insbesondere Methoxy.

Die erfindungsgemäße Reaktion kann in Gegenwart oder Abwesenheit von Verdünnungsmitteln durchgeführt werden. Als Verdünnungsmittel kommen alle organischen Lösungsmittel in Frage, in welchen sich die Reaktionspartner lösen und die selbst nicht an der Reaktion teilnehmen. Beispielhaft seien genannt Alkohole wie Methanol, Ethanol, Benzol, Toluol.

Die Reaktionstemperaturen können in einem größeren Bereich variiert werden. Im allgemeinen arbeitet man etwa zwischen 50 und 250°C, vorzugsweise zwischen 100 und 180°C.

Die als Ausgangsverbindungen eingesetzten Pyrrol-Derivate der allgemeinen Formel (II) stellt man her analog der Vorschrift von H.J. Roth et al. (EP 5205 vom 17.4.79).

Die Umsetzung der Aminocyanopyrrole zu Verbindungen der allgemeinen Formel (IV) und (V) erfolgt nach einer Vorschrift von W. Zimmermann, K. Eger und H.J. Roth, Arch. Pharm. (Weinheim) 309, 597 (1976).

Die Decarboxylierung der Pyrrolo[2,3-b]pyridin-dicarbonsäuren der allgemeinen Formel (VII) mit Phosphorsäure erfolgt nach einer Variante der Vorschrift von J. Chu und B.M. Lynch, J.Med.Chem. 18, 164 (1975).

Zur vorliegenden Erfindung gehören pharmazeutische Zubereitungen, die neben nicht-toxischen inerten pharmazeutisch geeigneten Trägerstoffen einen oder mehrere erfindungsgemäße Wirkstoffe enthalten, oder die aus einem oder mehreren erfindungsgemäßen Wirkstoffen bestehen, sowie Verfahren zu ihrer Herstellung.

Zur vorliegenden Erfindung gehören auch pharmazeutische Zubereitungen in Dosierungseinheiten. Das bedeutet, daß die Zubereitungen in Form einzelner Teile (z.B. Tabletten, Dragees, Kapseln, Pillen) vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Ampullen, Tabletten, Dragees, Kapseln, Pillen, Granulate, Lösungen, Emulsionen und Suspensionen genannt. Die Tabletten, Dragees, Kapseln, Pillen und Granulate können den oder die Wirkstoffe neben den üblichen Trägerstoffen enthalten, wie Füll- und Streckmittel

(z.B. Stärken, Milchzucker, Rohrzucker, Glukose, Mannit, Kieselsäure), Bindemittel (z.B. Carboxymethylcellulose, Alginate, Gelatine, Polyvinylpyrrolidon), Feuchthaltemittel (z.B. Glycerin), Sprengmittel (z.B. Agar-Agar, Calciumcarbonat, Natriumcarbonat), Netzmittel (z.B. Cetylalkohol, Glycerinmonostearat), Adsorptionsmittel (z.B. Kaolin, Bentonit) und Gleitmittel (z.B. Talkum, Calcium- und Magnesiumstearat, feste Polyethylenglykole). Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Der oder die Wirkstoffe können gegebenenfalls mit einem oder mehreren der oben angegebenen Trägerstoffe auch in mikroverkapselter Form vorliegen. Sie können, falls erforderlich, mit magensaftresistenten Umhüllungen versehen sein.

Die therapeutisch wirksamen Verbindungen sollen in den oben aufgeführten pharmazeutischen Zubereitungen vorzugsweise in einer Dosierung von 5 bis 500 mg, insbesondere 50 bis 150 mg, enthalten sein.

Lösungen und Emulsionen können neben dem oder den Wirkstoffen die üblichen Trägerstoffe, wie Lösungsmittel und Lösungsvermittler, enthalten. Als Lösungsmittel

können insbesondere Wasser oder niedere Alkohole sowie niedere Glykole, wie Propylenglykol und 1,3-Butylenglykol, in Frage kommen.

Als Lösungsvermittler können Verbindungen aus der Reihe der Polyglykole dienen, welche noch mit Ölen, insbesondere Baumwollsaatöl, Erdnußöl, Maiskernöl, Olivenöl, Rizinusöl, Sesamöl oder aber Glycerin vermischt sein können.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel (z.B. Wasser, Ethanol, Propylenglykol) und Suspendiermittel (z.B. ethoxylierte Isostearylalkohole, polyethoxylierte Isostearylalkohole, Polyoxyethylensorbit, Agar-Agar und Tragant oder Gemische dieser Stoffe) enthalten.

Zur parenteralen Applikation sind Lösungen vorgesehen, welche pro Ampulle 5 bis 500 mg, insbesondere 10 bis 100 mg, der therapeutisch wirksamen Verbindungen enthalten sollen.

Die Herstellung der oben angeführten pharmazeutischen Zubereitungen erfolgt in üblicher Weise und nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, insbesondere oral und intravenös, appliziert werden.

Im allgemeinen hat es sich als vorteilhaft erwiesen, den oder die Wirkstoffe bei parenteraler (i.v. oder i.m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 30, insbesondere 0,03 bis 3 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die tierexperimentelle Prüfung der erfindungsgemäßen Verbindungen ergab, daß ein Teil dieser Substanzen sedative und antikonvulsive Eigenschaften besitzt. Darüber hinaus führen sie auch zu einer Hemmung der Entzündung, gemessen an der Beeinflussen des Pfotenödems der Ratte nach Kaolininjektion.

Bei oraler Applikation zeigen die erfindungsgemäßen Verbindungen eine signifikante entzündungshemmende Wirkung am Kaolinödem der Rattenpfote nach Gaben von 25 mg/kg. Bei intramuskulärer Applikation zeigen die Verbindungen bereits bei Dosierung von 10 mg/kg eine deutliche Hemmung der Entzündung. Für die Verbindung des Beispiels 1 beträgt die $DE_{50}$ bei oraler Applikation 68 mg/kg und die $DE_{50}$ bei intramuskulärer Applikation 0,9 mg/kg.

Das folgende generelle Beispiel soll das Verfahren zur Herstellung der erfindungsgemäßen Verbindungen noch näher erläutern. Die darauf folgenden Beispiele 1 bis 11 wurden ähnlich durchgeführt.

Ausführungsbeispiele

Synthese der 1-substituierten 3-Cyano-4,5-dimethyl-pyrrole-2-yl-amino-maleinsäurediethylester
(Allgemeine Formel IV, wenn $R_4$ = Ethoxycarbonyl)

(0,1 mmol) 1-substituierte 2-Amino-3-cyano-4,5-dimethyl-pyrrole (II) und (0,1 mmol) Oxalessigester (III) werden in 150 ml Benzol mit 0,1 g p-Toluolsulfonsäure als Katalysator solange am Wasserausscheider erhitzt, bis sich die berechnete Menge Wasser abgeschieden hat. Anschließend wird das Lösungsmittel im Vakuum entfernt. Das angefallene Öl wird mit einer kleinen Menge 50 % Ethanol und Wasser versetzt und zur Kristallisation gebracht. Die angefallenen Kristalle werden abgesaugt, mit Petrolether gewaschen und aus Ethanol umkristallisiert.
Gelbgefärbte Kristalle, löslich in Ethanol, Aceton, DMSO, Methanol.
Schwer löslich in Petrolether, Hexan und Wasser.

Synthese der 7-substituierten 4-Amino-5,6-dimethylpyrrolo[2,3-b]pyridin-2,3-dicarbonsäureethylester
(Allgemeine Formel V, wenn $R_4$ = Ethoxycarbonyl)

0,1 mmol der 1-substituierten 3-Cyano-4,5-dimethyl-pyrrol-2yl-aminomaleinsäurediethylester und eine Lösung von (0,1 mmol) Natrium in 100 ml wasserfreiem Ethanol werden 2-6 Stunden erhitzt. Nach dem Erkalten werden die ausgefallenen Kristalle abgesaugt, mit Ethanol und Ether nachgewaschen und aus Ethanol oder DMF umkristallisiert.
Farblose, bis schwach gelb gefärbte Kristallbüschel.
Löslich in DMF, DMSO.
Schwer löslich in Petrolether, Ethanol.

Synthese der 7-substituierten 4-Amino-5,6-dimethyl-pyrrolo[2,3-b]pyridin-2,3-dicarbonsäuren
(Allgemeine Formel VI, wenn $R_5$ = Carboxyl)

(0,1 mmol) der 7-substituierten 4-Amino-5,6-dimethyl-pyrrolo[2,3-b]pyridin-2,3-dicarbonsäurediethylester werden mit 2 ml 50 % ethanolischer Kalilauge, 4 ml Wasser und 10 ml Ethanol 5 Stunden gekocht. Der abgekühlten Lösung wird tropfenweise eine 10%ige Salzsäurelösung zugetropft. Am isoelektrischen Punkt, bei pH 5-6, fallen gelbe, wollige Kristalle aus.
Gelbe, wollige Kristallbüschel
löslich in DMF, DMSO, Laugen und Säuren.
Schwer löslich in Petrolether, Ethanol.

Synthese der 7-substituierten 4-Amino-5,6-dimethyl-pyrrolo [2,3-b] pyridine
(Allgemeine Formel I, wenn $R_1$ = H)

0,1 mmol der 7-substituierten 4-Amino-5,6-dimethyl-pyrrolo [2,3-b] pyridin-2,3-dicarbonsäure werden mit 150 ml fünfundachtzigprozentiger Phosphorsäure 48 Stunden am Rückflußkühler erhitzt. Die noch heiße Lösung wird auf Eis gegossen, mit Ammoniak neutralisiert. Die ausgefallenen Kristalle werden abgesaugt, mit heißem Wasser nachgewaschen und aus Toluol umkristallisiert.
Löslich in Ethanol, DMSO, Aceton, DMF.
Schlecht löslich in Toluol, Hexan.

Beispiel 1

4-Amino-5,6-dimethyl-7-phenyl-pyrrolo[2,3-b]pyridin

Beispiel 2

4-Amino-5,6-dimethyl-7-(2'-fluorphenyl)-pyrrolo[2,3-b]-pyridin

Beispiel 3

4-Amino-5,6-dimethyl-7-(2'-chlorphenyl)-pyrrolo[2,3-b]-pyridin

Beispiel 4

4-Amino-5,6-dimethyl-7-(2'-bromphenyl)-pyrrolo[2,3-b]-pyridin

Beispiel 5

4-Amino-5,6-dimethyl-7-(2'-methylphenyl)-pyrrolo[2,3-b]-pyridin

| Beispiel-Nr. Summenformel MG | Schmp. °C Ausbeute (% d. Th.) | ber.: gef.: | C | H | N |
|---|---|---|---|---|---|
| 1 $C_{15}H_{15}N_3$ (237) | 120-122 (Toluol) 30,7 | | 75,94 75,35 | 6,32 5,92 | 17,72 17,05 |
| 2 $C_{15}H_{14}N_3F$ (255) | 155-157 (Ethanol) 45,7 | | 70,31 71,18 | 5,85 5,65 | 16,40 16,03 |
| 3 $C_{15}H_{14}N_3Cl$ (271) | 168-171 (Ethanol) 66,5 | | 66,42 66,35 | 5,16 5,02 | 15,49 14,89 |
| 4 $C_{15}H_{14}N_3Br$ (316) | 152-154 29,5 (Ethanol) | | 56,96 57,37 | 4,43 4,30 | 13,29 13,56 |
| 5 $C_{16}H_{17}N_3$ (251) | 134-136 (Ethanol) 24,6 | | 76,49 76,14 | 6,77 6,57 | 16,73 16,03 |

Beispiel 6

4-Amino-5,6-dimethyl-7-(4'-fluorphenyl)-pyrrolo[2,3-b]-pyridin

Beispiel 7

4-Amino-5,6-dimethyl-7-(4'-chlorphenyl)-pyrrolo[2,3-b]-pyridin

Beispiel 8

4-Amino-5,6-dimethyl-7-(4'-bromphenyl)-pyrrolo[2,3-b]-pyridin

Beispiel 9

4-Amino-5,6-dimethyl-7-(4'-methylphenyl)-pyrrolo[2,3-b]-pyridin

Beispiel 10

4-Amino-5,6-dimethyl-7-(4'-methoxyphenyl)-pyrrolo[2,3-b]-pyridin

| Beispiel-Nr. Summenformel MG | Schmp. °C Ausbeute (% d. Th.) | ber.: gef.: | C | H | N |
|---|---|---|---|---|---|
| 6 | | | | | |
| $C_{15}H_{14}N_3F$ (255) | 170-172 (Toluol) 27,9 | | 70,31 70,47 | 5,85 5,54 | 16,40 16,46 |
| 7 | | | | | |
| $C_{15}H_{14}N_3Cl$ (271) | 142-144 (Ethanol) 21,2 | | 66,29 65,97 | 5,15 5,28 | 15,46 15,29 |
| 8 | | | | | |
| $C_{15}H_{14}N_3Br$ (316) | 176-178 (Ethanol) 32,0 | | 56,96 56,4 | 4,43 4,32 | 13,29 14,90 |
| 9 | | | | | |
| $C_{16}H_{17}N_3$ (251) | 135-137 (Ethanol) 41,5 | | 74,49 77,0 | 6,77 6,45 | 16,73 15,7 |
| 10 | | | | | |
| $C_{16}H_{17}N_3O$ (267) | 98-100 (Toluol) 32,5 | | 72,85 71,9 | 7,14 7,0 | 20,0 19,5 |

TP 39

Beispiel 11

4-Amino-5,6-dimethyl-7-(4'-dimethylaminophenyl)-pyrrolo-[2,3-b]-pyridin.

| Beisp.-Nr.<br>Summenformel<br>MG | Schmp. °C<br>Ausbeute (% d.Th.) | Ber.:<br>Gef.: | C | H | N |
|---|---|---|---|---|---|
| 11<br>$C_{17}H_{20}N_4$ (280) | 95-97 (Toluol)<br>32,5 | | 72,85<br>72,18 | 7,14<br>7,04 | 20,0<br>18,1 |

Patentansprüche

1) 5,6-Dimethyl-pyrrolo/2,3-b/-pyridine der allgemeinen Formel (I)

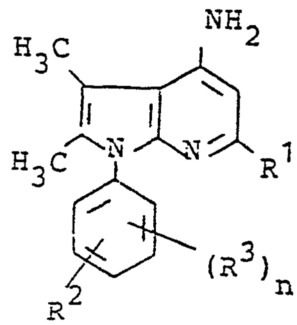

(I)

in welcher

$R_1$ für Wasserstoff oder niederes Alkyl steht,

$R_2$ für Wasserstoff, Halogen, Nitro, niederes Alkyl oder Alkoxy, Trifluormethyl oder Dimethylamino steht,

$R_3$ jeweils für Wasserstoff, Halogen oder niederes Alkyl oder Alkoxy steht und

n für 1 oder 2 steht,

sowie ihre physiologisch unbedenklichen Additionssalze.

2) Verbindungen der allgemeinen Formel (I) gemäß Anspruch 1, in welcher

$R_1$ für Wasserstoff oder Methyl steht,

$R_2$ für Wasserstoff, Fluor, Chlor, Brom, Nitro, Trifluormethyl, Alkyl oder Alkoxy mit je 1 bis 2 Kohlenstoffatomen steht,

$R_3$ für Wasserstoff, Fluor, Chlor, Brom, Methoxy oder Methyl steht und

n für 1 oder 2 steht,

sowie ihre physiologisch verträglichen Additionssalze.

3) Verfahren zur Herstellung von Verbindungen der allgemeinen Formel (I) gemäß Anspruch 1, dadurch gekennzeichnet, daß man Pyrrolderivate der allgemeinen Formel (II)

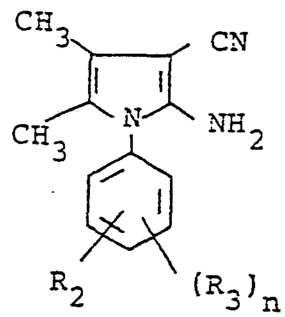

(II)

mit Diketoverbindungen der allgemeinen Formel (III)

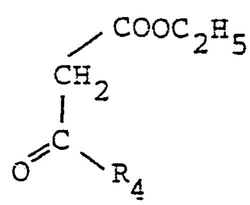

(III)

in welcher

$R_4$ für die Methyl- oder die Ethoxycarbonylgruppe steht,

in Gegenwart von p-Toluolsulfonsäure als Katalysator und in Gegenwart von inert-organischen Lösungsmitteln bei erhöhter Temperatur zu Verbindungen der allgemeinen Formel (IV) umsetzt,

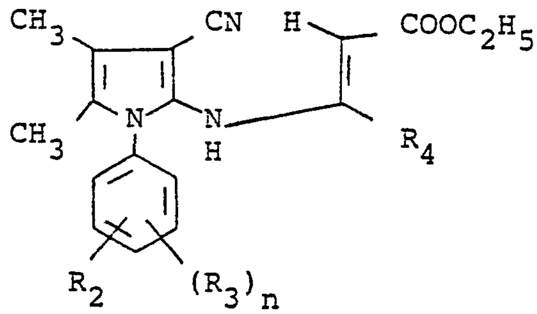

(IV)

in welcher
$R_2$,$R_3$,$R_4$ und n die oben angeführte Bedeutung haben,

und die Verbindung der allgemeinen Formel (IV) anschließend in Gegenwart von organischen Lösungsmitteln und gegebenenfalls in Gegenwart von Katalysatoren bei erhöhter Temperatur zu Verbindungen der Formel (V)

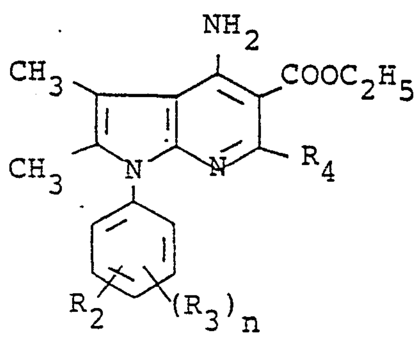

(V)

cyclisiert und diese Verbindungen anschließend in Gegenwart von alkoholischer Kalilauge und gegebenenfalls in Gegenwart von inerten Lösungsmitteln bei erhöhter Temperatur und anschließender Zufügung von Säuren zu Verbindungen der allgemeinen Formel (VI)

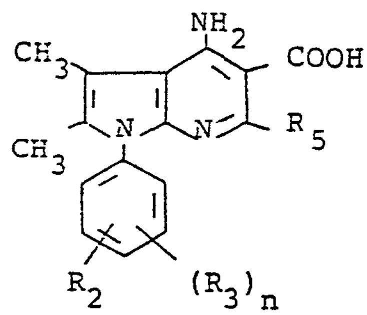

(VI)

in welcher

$R_2$, $R_3$ und n die oben angegebene Bedeutung haben,

$R_5$ für eine Methyl- oder Carboxylgruppe steht,

umsetzt, und anschließend die Verbindungen der allgemeinen Formel (VI) in Gegenwart von 85 %iger Phosphorsäure über einen kürzeren Zeitraum bei erhöhter Temperatur zu Verbindungen der allgemeinen Formel (VII)

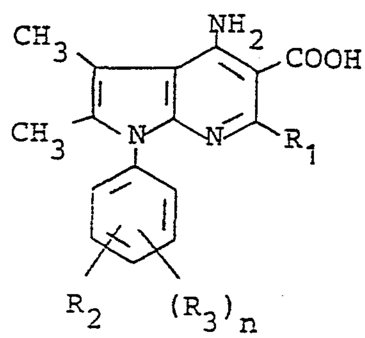

(VII)

in welcher

$R_1$, $R_2$, $R_3$ und n die oben angegebene Bedeutung haben,

umsetzt und diese Verbindungen der allgemeinen Formel (VII) dann in Gegenwart von 85 %iger Phosphorsäure über einen längeren Zeitraum bei erhöhter Temperatur zu Verbindungen der allgemeinen Formel (I) decarboxyliert.

4) Verbindungen der allgemeinen Formel (I) gemäß Anspruch 1 zur Verwendung bei der Bekämpfung von Erkrankungen.

5) Arzneimittel enthaltend mindestens eine Verbindung der allgemeinen Formel (I) gemäß Anspruch 1.

6) Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

7) Verwendung von Verbindungen der allgemeinen Formel (I) gemäß Anspruch 1 zur Bekämpfung von Erkrankungen.

8) Verwendung von Verbindungen der allgemeinen Formel (I) gemäß Anspruch 1 zur Bekämpfung von entzündlichen Prozessen.

9) Verwendung von Verbindungen der allgemeinen Formel (I) gemäß Anspruch 1 zur Bekämpfung von Erkrankungen des zentralen Nervensystems.

Europäisches Patentamt

# EUROPÄISCHER RECHERCHENBERICHT

0061056

Nummer der Anmeldung

EP 82 10 1812

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 005 205 (TROPONWERKE)<br>* Ansprüche 1,6 *<br>----- | 1,5 | C 07 D 471/04<br>A 61 K 31/44//<br>C 07 D 207/40<br>(C 07 D 471/04,<br>C 07 D 221/00,<br>C 07 D 209/00) |
| | | | RECHERCHIERTE SACHGEBIETE (Int. Cl. 3) |
| | | | C 09 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 03-06-1982 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument